# EUROPEAN PATENT SPECIFICATION

(11) **EP 4 410 183 B1**
(45) Date of publication and mention of the grant of the patent: **01.04.2026**
(21) Application number: 22835856.0
(22) Date of filing: 30.09.2022
(51) Int. Cl.: A61B 5/00, A61B 5/103

(54) **SYSTEM FOR DIAGNOSING BALANCE, AND SENSOR-EQUIPPED PLATFORM OF SAID SYSTEM**
INSTALACIÓN PARA EL DIAGNÓSTICO DEL EQUILIBRIO Y PLATAFORMA SENSORIZADA DE DICHA INSTALACIÓN
INSTALLATION POUR LE DIAGNOSTIC DE L'ÉQUILIBRE ET PLATEFORME À CAPTEURS DE LADITE INSTALLATION

(30) Priority: 30.09.2021 ES 202130917
(43) Date of publication of application: 07.08.2024
(73) Proprietor: Universidad del País Vasco/Euskal Herriko Unibertsitatea, 48940 Leioa, Vizcaya (ES); Administración General De La Communidad Autónoma De Euskadi, 01010 San Sebastián (Araba/Álava) (ES)
(72) Inventor: PINTO, Charles, 48940 Leioa (ES); HERRERO VILLALIBRE, Saioa, 48940 Leioa (ES); CORRAL SAIZ, Javier, 48940 Leioa (ES); DIEZ SÁNCHEZ, Mikel, 48940 Leioa (ES); URÍZAR ARANA, Mónica, 48940 Leioa (ES); MACHO MIER, Erik, 48940 Leioa (ES); CAMPA GÓMEZ, Francisco Javier, 48940 Leioa (ES); TEJADA EZQUERRO, Pedro Ignacio, 01010 Vitoria-Gasteiz (ES); GARCÍA ORTIZ, María Mar, 01010 Vitoria-Gasteiz (ES); LÓPEZ MARTÍNEZ, Héctor, 01010 Vitoria-Gasteiz (ES)
(74) Representative: Balder IP Law, S.L.
(86) International application number: PCT/ES2022/070622
(87) International publication number: WO 2023/052670

(56) References cited:
- CN-A- 111 419 627
- KR-A- 20180 102 153
- US-A1- 2015 328 497
- KHARBOUTLY HAISSAM ET AL: "Design of Multiple Axis Robotic Platform for Postural Stability Analysis", IEEE TRANSACTIONS ON NEURAL SYSTEMS AND REHABILITATION ENGINEERING, IEEE, USA, vol. 23, no. 1, 1 January 2015 (2015-01-01), pages 93 - 103, XP011569341, ISSN: 1534-4320, [retrieved on 20150106], DOI: 10.1109/TNSRE.2014.2329533

## Description

### TECHNICAL FIELD

The present invention relates to a system for the diagnosis and rehabilitation of patients/users with balance deficiencies or who want to improve their balance. In systems of this type, the patient gets onto the platform of the system, and the system performs movements that may be previously programmed, during which data that provides information about the status of the patient's balance function, such as the position of the patient's center of pressure on the platform, or the number of times and the position in which he or she has had to hold on to the safety handles to regain his or her balance, as well as the grip force exerted, is recorded. The system also allows the generation of accessible data through a USB connection, and such data can be provided to the doctor, physical therapist or rehabilitation therapist who has a program for processing it.

### BACKGROUND

The state of the art comprises systems that are capable of generating certain movements on a platform in order to achieve different objectives. US3645011 describes one of these systems where, by means of a front hydraulic cylinder and two rear hydraulic cylinders, said platform can be rotated according to both an anteroposterior movement and a lateral movement, the resulting rotation being made up of said anteroposterior and lateral rotations. Mounted on the platform is a cabin in which a person can be placed to perceive the movement of the platform as if it were the movement of an airplane that he or she was piloting.

The state of the art also comprises systems that generate movement for therapeutic purposes. One of these systems is described in publication US6162189A. In one of the embodiments, the system has two mobile platforms attached to two fixed platforms by means of pneumatic actuators. Each of the mobile platforms can move with respect to the fixed platforms in the directions defined by three perpendicular Cartesian axes and rotate around each of these axes such that it can move with 6 degrees of freedom. Each of these platforms has a fastening element for each of a person's feet. Said fastening elements are provided with force sensors. A controller controls the movement of the pneumatic actuators in order to control the movement of the mobile platforms. By means of force sensors located in the clamping elements, the forces exerted by the patients' feet in response to the movement of the mobile platforms are measured. The movement to which the patients' feet are subjected is beneficial for recovering from injuries to the lateral ligaments of the patients' ankles. In an embodiment of the invention described in US6162189A, the movement of the platforms is controlled so that the patient can exercise his or her balance. US6162189A additionally describes the possibility of recording the patient's movements by means of a camera, and it also describes that the system can have screens where patients can see images of the movement they are making. Although the system described in US6162189A provides an enormous capacity for movement of the mobile platforms, it is only designed to measure forces in the patients' feet, which feet are fastened to the mobile platforms by means of fastening elements that prevent the movement of each foot with respect to the corresponding mobile platform.

The control of each mobile platform is relatively complicated, as it is necessary to control the movement of 6 actuators to define its movement.

There is therefore a need for a system suitable for measuring and exercising the movement of a patient in which, by means of the movement of a platform controlled by a reduced number of actuators, the change in position of the patient's feet and the force exerted by same when the patient/user tries to maintain his or her balance can be measured. In addition, it would be desirable to be able to measure not only the movement of the patient's feet but also the movement of his or her entire body in order to measure and exercise his or her balance with extreme precision.

US2015/328497A1 discloses a virtually-interfaced robotic ankle & balance trainer. KR20180102153A discloses a mobile platform for physical exercise. CN111419627A discloses a four-degree-of-freedom testing device for dynamic balance ability of human body under electrical stimulation and a method related to the device. Kharboutly Haissam et al. "Design of Multiple Axis Robotic Platform for Postural Stability Analysis", IEEE TRANSACTIONS ON NEURAL SYSTEMS AND REHABILITATION ENGINEERING, IEEE, USA, vol. 23, no 1, January 2015 (2015-01-01), pages 93-103, XP011569341 discloses a robotic platform for analysis of postural stability, wherein the robotic platform is a multiple axis platform.

### DESCRIPTION OF THE INVENTION

The aforementioned technical problems are solved by means of a system for diagnosing the balance of a user according to claim 1.

Other advantageous embodiments are embodiments according to the dependent claims. Claim 5 sets out a particular embodiment of the invention.

The system for diagnosing the balance of a user of the invention comprises a sensor-equipped platform and a U-shaped support structure that partially surrounds the platform such that the user has sufficient space to access the platform. The system has guiding elements for the mobile ends of three linear actuators that allow the vertical displacement of said mobile ends of the actuators with respect to the structure. Said mobile ends are mechanically connected to the platform such that height and inclination of the platform are adjustable by vertical displacement of said mobile ends. The system has the advantage that by controlling the vertical movement of the ends of the actuators, the movement of the platform is controlled in a relatively simple way.

In an advantageous embodiment of the invention, the platform can descend until its lower surface is substantially in the same plane as the lower surface of the support structure. This fact facilitates user access to the platform.

In a preferred embodiment, one of the linear actuators is arranged on the side opposite the access to the platform defined by the U-shaped support structure, with the two other linear actuators arranged on opposite sides of said U-shaped structure, close to the free ends of said opposite sides of said U-shaped structure, such that the actuators are preferably arranged at the vertices of an imaginary triangle the sides of which join the three linear actuators together. This arrangement is advantageous since the movement of the linear actuator arranged on the side opposite the access to the platform causes the anteroposterior rotation of the platform and, similarly, the movement of the actuators arranged on opposite sides of said U-shaped structure cause the lateral rotation of the platform. The final rotation of the platform is made up of these two rotations which, as indicated, are decoupled such that the total rotation control of the platform is simplified.

In the most frequent embodiment of the invention, the platform is attached to the mobile ends of the actuators by means of bars. Preferably, the bars are attached to the mobile ends of the actuators by means of cylindrical joints and to the platform by means of ball-and-socket joints.

The system contains said bars, said cylindrical actuators, said ball-and-socket joints and the platform that can rotate laterally with respect to an instantaneous axis of rotation contained in said platform and that passes through the ball-and-socket joint (R1) located on the side opposite the access defined by the U-shaped platform and a point belonging to said platform that is at the same distance from the ball-and-socket joints (R2, R3) located close to the free ends of the U-shaped structure.

The platform can also perform an anteroposterior rotation when the bar B1 and the ball-and-socket joint R1 that is integral with it rotate with respect to the joint C1. Said joint preferably has as its only degree of freedom the rotation of the bar B1 with respect to same. When the bar B1 and the ball-and-socket joint R1 that is integral with it rotate with respect to the joint C1, the platform rotates with respect to an axis contained in it and containing the ball-and-socket joints R2, R3.

The movement indicated above results in the platform also moving vertically, although when it performs this movement not all its points are at the same height with respect to the base of the U-shaped structure.

Preferably, each of the actuators is made up of a motor that moves a worm screw which, upon rotation, moves the free end of each actuator vertically.

Preferably, the joints (C1, C2, C3) allow the bars (B1, B2, B3) to rotate with respect to imaginary axes of rotation that pass through said joints and are parallel to the lower support surface.

In the most common case, the sensor-equipped platform comprises force sensors.

In order to help maintain balance during the exercise/measurement of the user's balance, the system has sensor-equipped supports in the form of handles such that the user can hold on to them during the exercise.

In an advantageous embodiment of the invention, the system has a central sensor-equipped support in the form a handle and two sensor-equipped supports in the form of handles placed laterally with respect to the central support.

Each of these sensor-equipped supports in the form of handles can have three force sensors.

In order to protect the support structure, a U-shaped outer casing surrounds the support structure.

In order for the user to be able to lean on, hold on to the sensor-equipped supports in the form of handles, such handles can be arranged in the upper part of the part of the casing opposite the entrance defined by the U-shaped structure.

According to the invention, the sensor-equipped platform is adapted to be used in one of the aforementioned systems and is formed by a first upper plate and a second lower plate, the upper plate being superimposed on the lower plate, with force sensors arranged between the upper plate and the lower plate.

Preferably, said sensors are single-axis sensors based on strain gauges that are preferably capable of measuring forces of up to 2000 N.

In order for the ball-and-socket joints that are arranged on the lower plate to be able to protrude from it without coming into contact with the upper plate and be able to be connected directly with the bars, the surface of the upper plate can be different from the surface of the lower plate.

### BRIEF DESCRIPTION OF THE DRAWINGS

To complement the description and for the purpose of aiding to better understand the features of the invention according to practical embodiments of the system of the invention, a set of figures is attached as an integral part of the description in which the following has been depicted with an illustrative and nonlimiting character:
Figure 1 is a perspective view of the system of the invention.
Figure 2 is another perspective view of the system, but without the external casing.
Figure 3 is a front view of the system with a user/patient on the platform in a standing position.
Figure 4 is a perspective view of the system with a user/patient on the platform.
Figure 5 is a plan view of the system with a patient/user on the platform.
Figure 6a is the plan view of the platform.
Figure 6b is the elevational view of the platform.
Figure 7a is a profile view of the platform.
Figure 7b is the plan view of the lower part of the platform.
Figure 8 is a front view of the system.
Figure 9 is the plan view of the system, including the platform.
Figure 10 is the plan view of the system in which the bar can be seen as a U-shaped railing.
Figure 11 is a profile view of the system.

### DESCRIPTION OF EMBODIMENTS OF THE INVENTION

The present invention relates to a system for the diagnosis and rehabilitation of patients/users with balance deficiencies or who want to improve their balance.

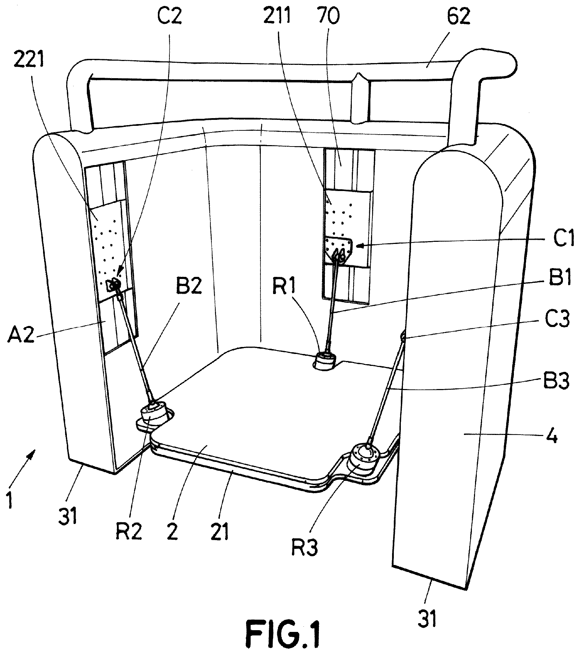

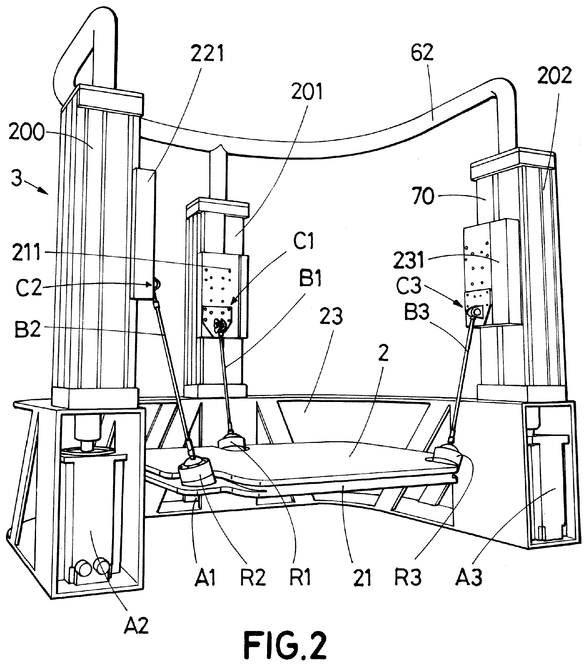

As can be seen in Figures 1 and 2, the system comprises a substantially rectangular platform (2, 21) intended to support a user, a support structure (3) and at least three vertical linear actuators (A1, A2, A3) connected to the platform (2, 21).

The structure comprises a base (31), an inner metallic wall (23) that surrounds the platform (Figure 2) and three vertical columns (200, 201, 202) preferably located so as to define an isosceles triangle. The structure comprises a bar in the form of a U-shaped railing (62) in its upper part. As can be seen in Figure 1, the support structure is surrounded by an also U-shaped fairing (4). Both the structure and the fairing partially surround said platform and define an inner space where the platform (2, 21) is located. In an advantageous embodiment, the fairing (4) is made up of mixed methacrylate-aluminum elements.

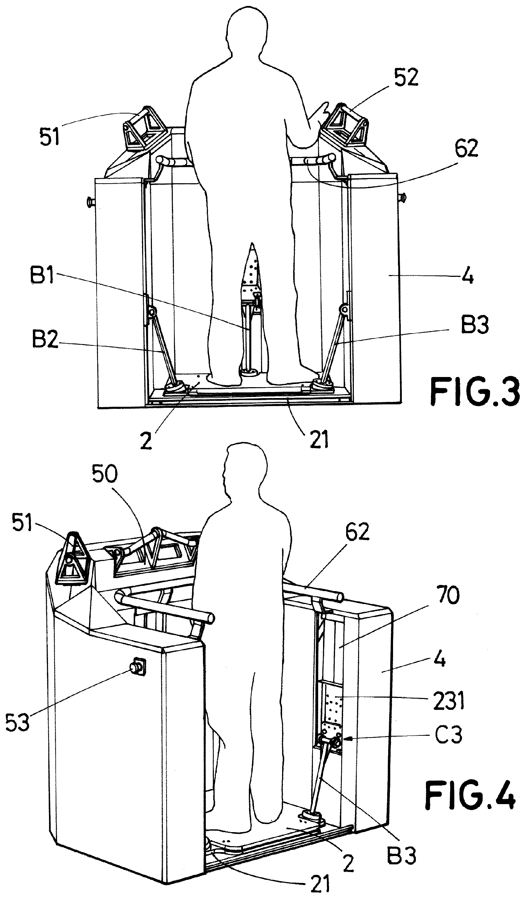

As illustrated in Figures 1 and 2, the bar in the form of a U-shaped handrail (62) preferably has a tubular shape, is not sensor-equipped and serves both for positioning the patient when getting on or off the platform and as a gripping point for patients in case they feel that they are losing their balance with the risk of falling. The position of this bar is also illustrated in Figure 4.

Preferably, each of the vertical linear actuators (A1, A2, A3) is formed by a motor that rotates a worm screw extending vertically along the inside of the support structure (3). Upon rotation, said worm screw moves a mobile end integral with a joint arranged in the upper part of the corresponding bar (B1, B2, B3) that attaches said joint to the platform (2, 21). (see Figure 2).

In an alternative embodiment, in the event that the loads sustained by the platform are not excessive, the motor and the worm screw can be replaced with a linear motor.

In one embodiment of the invention, the worm screw has a stroke of 400 mm and is made of steel.

The platform (2, 21) is preferably formed by two plates located one above the other as illustrated in Figures (1, 3, 4). In order to lighten a platform that serves to transmit movement to a user who is placed on it in a standing position (Figure 3), the platform is preferably made of aluminium. The support structure (3) partially surrounds the platform (2) so as to define, as illustrated in Figure 1, an entry side for a user.

Figure 1 illustrates that the structure (3) comprises a lower support surface (31) and, as indicated above, an upper U-shaped railing (62) open on the entry side for the user, the lowest position of the platform 2 being a position in which its lower surface is at the level of the lower support surface 31 or substantially at the level of the lower support surface of the support structure 3. The system thereby provides comfort and safety to users who are to be subjected to the biomechanical diagnostic test.

Due to this configuration, the user/patient can be placed on top of the platform without hindrance when subjected to a balance test. As can be seen in Figure 3, once the user stands on the platform, he or she is in a standing position.

Figure 2 illustrates that one of the actuators (A1) is arranged on the side opposite the entrance defined by the support structure (3), the other two linear actuators (A2, A3) being arranged on the sides, close to the free ends of the fairing (4) such that an actuator (A1) is arranged on the side of the platform opposite the entrance and the other two (A2, A3) opposite respective inner parts of the sides of the casing that are closest to the entrance.

The linear actuators (A1, A2, A3) have mobile ends (211, 221, 231) integral with cylindrical joints (C1, C2, C3) that articulate a first end of the bars (B1, B2, B3). A second end of the bars opposite the first end is attached to the platform (2, 21 Figure 1), in particular to its lower plate (21), by means of ball-and-socket joints (R1, R2, R3). The movement of each of the linear actuators (A1, A2, A3) is thereby transmitted to the platform.

The cylindrical joints (C1, C2, C3) have horizontal axes of rotation preferably in a plane parallel to the plane containing the lower support surface (31) of the structure (3).

The system for biomechanical diagnosis, in addition to generating movement in order to test the balance of the patient/user, is also designed to take measurements that will serve to evaluate said balance. These measurements are primarily position and force measurements. In order to be able to measure the position of the platform, the motors have rotation counters and the worm screws have position sensors. Rotation sensors are useful for determining the position of the mobile part (211, 221, 231) of linear actuators, since the rotations of each of the motors are converted into displacements of the mobile ends of the actuators (A1, A2, A3).

In a preferred embodiment, the platform is formed by two aluminum plates that can have a minimum thickness of 12 mm superimposed one on top of the other. The surface of the upper plate (2) differs from the surface of the lower plate (21) such that the bars (B1, B2, B3) joining the platform with the linear actuators can be directly attached to the lower plate (21) by ball-and-socket joints.

Superimposing said plates (2, 21) is advantageous since force sensors (40, 41, 42, 43) that allow the measurement and localization of the forces exerted by the user on the platform are arranged between both plates.

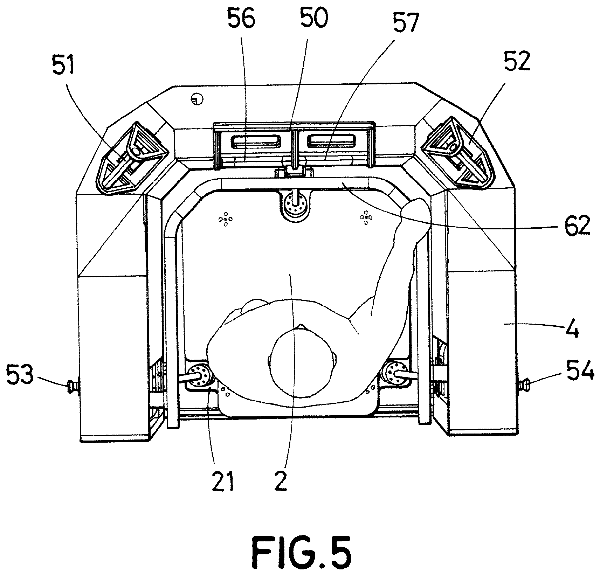

According to one embodiment, the lower aluminum plate (21) has a dimension of 800 x 800 mm. The upper plate has basically the same dimension with the only exception that the upper plate has recesses (see Figure 5) so that the bars can be directly connected to the lower plate by means of ball-and-socket joints.

In a preferred embodiment, the platform has force sensors (40, 41, 42, 43) based on strain gauges, preferably 4 force sensors, capable of measuring forces of up to 2000 N.

In order to be able to measure the force exerted by the patient's hands, the system has three sensor-equipped supports in the form of handles for the patient's hands, one at the front (50) and two at the sides (51, 52) on each side of the central support (Figure 5). In a preferred embodiment the supports are located at substantially 45 degrees from the central support. This arrangement is illustrated in Figure 5.

According to one embodiment of the invention, each of the handles has three single-axis force sensors based on strain gauges capable of measuring a force of up to 250 N.

Each of the ball-and-socket joints (R1, R2, R3) has three degrees of freedom corresponding to the possibility of rotation with respect to three imaginary perpendicular axes that intersect at a common vertex contained in said ball-and-socket joint. The ball-and-socket joints are each attached to a bar (B1, B2, B3) the first end of which is attached to the moving part of a linear actuator by means of a joint (C1, C2, C3). In this way, the height of each ball-and-socket joint (R1, R2, R3) with respect to the lower support surface (31) of the casing (4) can be adjusted. In addition to the aforementioned degrees of freedom of rotation, each of the ball-and-socket joints (R1, R2, R3) can move with the center of the corresponding joint (C1, C2, C3) and with a radius of rotation the length of the bar (B1, B2, B3) to which it is attached. In the preferred embodiment as mentioned above, this rotation is performed with respect to an imaginary axis of rotation that passes through said joint and is parallel to a plane that contains the lower support surface (31) of the structure (3).

As can be seen in Figures 1 and 2, the ball-and-socket joints are preferably attached to the platform (2, 21) in an arrangement corresponding to the vertices of an isosceles triangle.

In this arrangement, by controlling the height of the mobile elements (221, 231) of the linear actuators (A2, A3) that are located close to the free ends of the casing (4), the platform (2, 21) can be rotated laterally with respect to an instantaneous axis of rotation comprised in the platform and that passes through the ball-and-socket joint (R1) located on the side opposite the access to the platform (which ball-and-socket joint can also change position), which axis contains a point belonging to the platform and equidistant from the two ball-and-socket joints (R2, R3) arranged close to the free ends of the opposite sides of the U-shaped structure. Said axis of rotation is instantaneous since, as the position of the ball-and-socket joints (R1, R2, R3) changes over time, the aforementioned points contained in said axis also change over time. It is therefore possible throughout the exercise to rotate with respect to different axes contained in the platform, which not only rotates but also varies in height such that the possibilities of movement are great enough to be able to conscientiously test the balance of the patient. In the event that the platform moves only according to this lateral rotation, one of the ball-and-socket joints (R2, R3), e.g. R3, will rotate with respect to joint C3 while the opposite ball-and-socket joint (R2) will perform an opposite rotation with respect to joint C2.

As previously mentioned, the platform can also perform an anteroposterior rotation when the bar B1 and the ball-and-socket joint R1 that is integral with it rotate with respect to the joint C1. This movement causes a rotation of the platform with respect to an axis contained in said platform and containing the ball-and-socket joints R2, R3.

The platform can also perform a pure vertical movement when each of the ball-and-socket joints R1, R2, R3 is displaced in the vertical direction by each traveling the same distance.

The total movement of the platform is preferably made up of one or more of the lateral, anteroposterior and vertical movements described in the previous paragraphs.

The heights at which the free ends (211, 221, 231) of each of the linear actuators are located define the height of the platform and its inclination.

The maximum lateral angle of rotation of the platform with respect to the fairing is +-20 degrees, preferably +-15 degrees.

It is worth pointing out that the construction method of the system of the present invention is especially advantageous since it is only necessary to control the rotation of the motors of each of the three actuators (A1, A2, A3) to be able to rotate the platform laterally, anteroposteriorly, and regulate its height.

By controlling the rotation sequence of the motors of the actuators (A1, A2, A3), the ideal sequence of compound movements (lateral rotation, anteroposterior rotation, height displacement) in order to assess or train the balance of the patient or user can be defined. The ideal movement sequences can be programmed.

The system has a series of additional elements such as controllers of the motors and a PC panel that acts as an interface for the doctor, rehabilitation therapist or physical therapist, additional elements that are arranged in a control cabinet (not illustrated in the figures) attached to the fairing (4).

In order to increase the safety of the system, said cabinet contains and protects the electrical connection elements. Said cabinet is electrically connected with the motors of the actuators through an insulating hose (not illustrated in the figures). The system, see Figure (4), incorporates safety stop buttons (53, 54) which, if pressed, cut off the power supply to the motors of the actuators (A1, A2, A3), stopping the movement of the platform.

Based on the information provided by the platform's force sensors, the system uses a computer program to calculate in real time the user's center of pressure on it. In order for the doctor, rehabilitation therapist or physical therapist to be able to assess the patient's degree of balance, the system also collects the information provided by the sensors placed on the three sensor-equipped supports (50, 51, 52) for the user's hands, motor position and rotation sensors and the measurement of motor torque. The system uses a computer program to calculate in real time the position of the user's center of pressure. Said center of pressure can be viewed by the user on a screen (not illustrated in the figures) in which a reference center of pressure is also shown that serves to improve posture at all times of the balance test.

In the diagnostic phase, the doctor, rehabilitation therapist or physical therapist will be able to assess the status of the patient's balance function based on the measurements taken by the platform and the results of processing those measurements.

Once the status of the balance function has been established, the healthcare personnel, doctor, rehabilitation therapist or physical therapist will be able to design a training plan adapted to the patient and his or her needs. To help both in the diagnosis and in the design of a training plan, the healthcare personnel can extract the data recorded by the system through a USB port to which an external USB memory can be connected. The healthcare personnel can reprocess said data using a different computer program than the one used in the system.

In order to be able to evaluate the response of the patients to the movements of the platform, it is necessary for a series of healthy users to previously be subjected to the same movement sequence of the platform to which the patients are to be subjected. In this way it can be evaluated whether or not there is a deficiency in the balance of patients/users.

Given that certain movement sequences of the platform would cause even a healthy patient to lose their balance, endangering their health, the programming of platform movements is only possible after having entered an identification key.

Figure 3 illustrates the standing position of the user on the platform. This figure shows the position of the sensor-equipped supports in the form of right (52) and left (51) handles with reference to the direction of access to the platform. As previously mentioned, these supports serve so that when patients hold on to one or more of them during the exercise, they can regain balance in their position.

It can be seen in Figure 4 that the system has a sensor-equipped support (50) in the form of a central handle. In this figure, the position of the handle in the form of a U-shaped handrail (62) can be distinguished. Said handle (62) is in a position less distant from the lower support surface (31) than the sensor-equipped supports in the form of handles (50, 51, 52). In this way, if the patient loses his or her balance quickly and feels that he or she might fall, he or she can hold on to the handle in the form of a railing (62) in order to avoid an accident. As previously mentioned, the system has one or more emergency stop buttons (53, 54) located on the sides of the fairing. Figure 4 illustrates the position of an emergency stop button (53) on the outer part of one side of the fairing at a suitable height so that the person supervising the performance of the exercise can cut off the power supply to the motors of the actuators in the unlikely event of a malfunction of the system or in the event that the patient responds poorly to the movements of the platform (2, 21) and may sustain an accident. This can happen if the patient becomes dizzy. Figure 4 illustrates, similarly to Figure 1, the guiding element (70) of the structure along which the free end (231) of the actuator (A3) is displaced linearly.

Figure 5 is a plan view of the system where the position of the sensor-equipped supports in the form of handles (50, 51, 52) is observed. Each of these supports preferably comprises three position sensors. As can be seen in Figure 5, the central sensor-equipped support can be formed by two supports that contain a first (56) and a second (57) inclined sensor-equipped bar the upper parts of which converge towards a common central point. This arrangement enables the patient to grip the first inclined bar with one hand and the second inclined bar with the other. Figure 5 illustrates an embodiment in which the system comprises two emergency stop buttons (53, 54), each located on the outside of the lateral sides of the U-shaped structure.

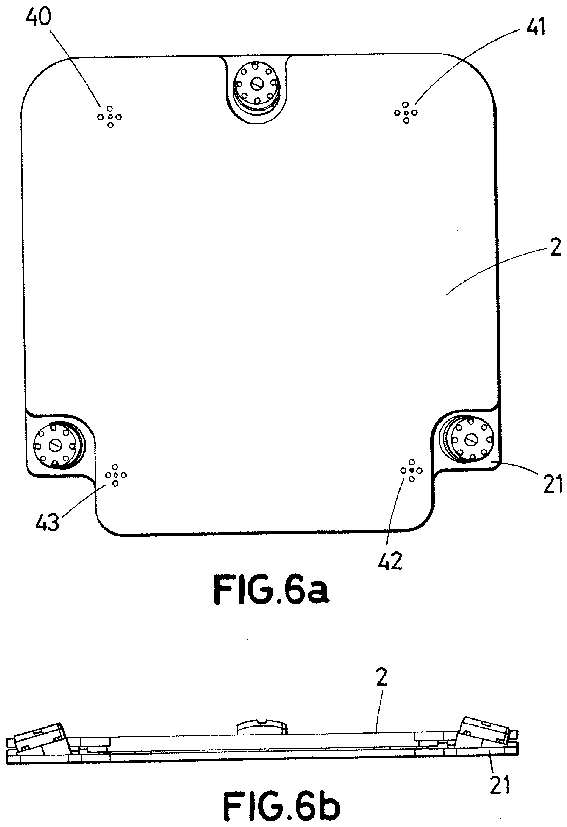

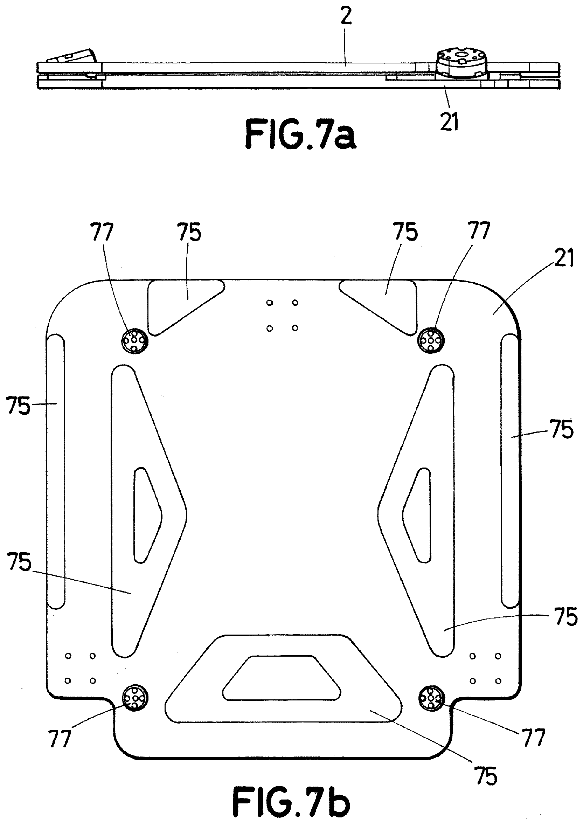

Figure 6a is a plan view of the mobile platform (2) formed by an upper plate (2) and a lower plate (21). The shape of the upper plate (2), which is designed so that, when the upper plate (2) is placed on the lower one plate (21) it does not cover its entire surface, but in three positions, which preferably define an isosceles triangle, ball-and-socket joints (R1, R2, R3), the lower part of which is fastened to the lower plate, protrude vertically above the plane containing the upper plate (21). This arrangement allows the bars (B1, B2, B3) to be mechanically connected directly to the lower plate, which is advantageous because when the lower plate (2) is displaced upwards, the two plates tend to come together, and when it is displaced downwards, the weight of the top plate helps the plates stay together. As previously mentioned, the construction of a platform by means of an upper plate (2) and a lower plate (21) makes it possible to place force sensors (40, 41, 42, 43) between the two plates, which sensors are preferably single-axis sensors based on strain gauges capable of measuring forces of up to 2000 N. In a preferred embodiment illustrated in Figures 6a, 6b and also in Figure 7b, the platform incorporates four sensors close to the corners of the platform, said sensors being located at the corners of an imaginary square the sides of which are defined by attaching adjacent sensors by lines. This arrangement in the form of square corners, which takes into account that the user/patient tends to stand in the center of the platform, makes it easier to calculate the center of pressure when subjected to the movement of the platform (2, 21).

Figure 7b illustrates that the lower plate of the platform has protrusions (75) that contact the upper plate when it is placed on the lower plate. Figure 7b also illustrates the housings where the force sensors (40, 41, 42, 43) are located.

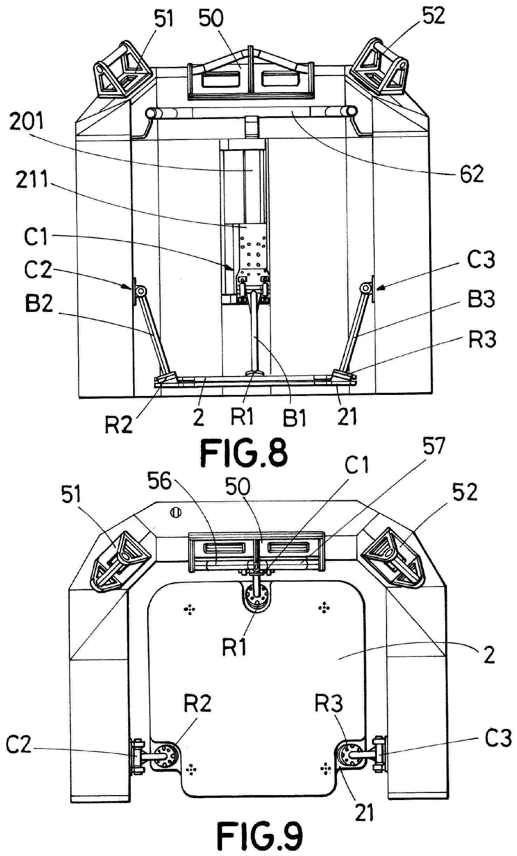

Figures 8, 9, 10, 11 describe elevational, profile and plan views of an embodiment of the platform. Figure 8 illustrates the elevational view of the platform. The arrangement of the ball-and-socket joints and the inclination of the bars (B1, B2, B3) that attach the joints (C1, C2, C3) with the ball-and-socket joints (R1, R2, R3) can be observed in this figure. This figure also illustrates the inclined arrangement of the parts (56, 57) of the sensor-equipped support in the form of a central bar (50). Said arrangement is ergonomically advantageous compared to a position parallel to the lower support surface (31) given the natural inclined position of the patient's/user's wrists with respect to his or her body.

Figure 9 shows the arrangement of the axes of rotation of the joints C1, C2, C3.

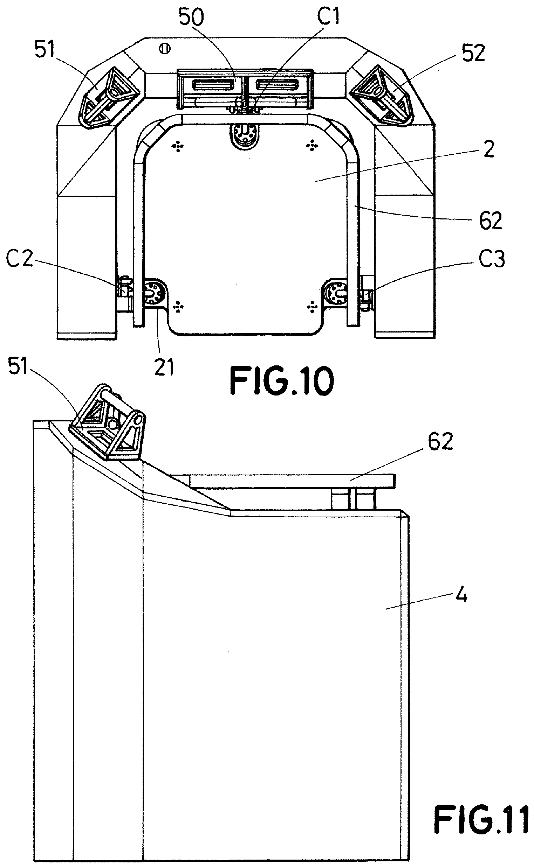

Figure 10 is a plan view of an embodiment of the system where the relative position of the platform (2, 21) with respect to the fairing can be seen. In the embodiment corresponding to this view, it is possible to see the joints (C1, C2, C3), which move vertically with the mobile elements of the actuators and allow the rotation of the bars (B1, B2, B3) with respect to them. As mentioned above, it can be seen in Figure 10 that in this embodiment the joints are arranged at the vertices of an isosceles triangle. Figure 10 also shows the non-sensor-equipped U-shaped railing (62).

Figure 11 is a profile view of an embodiment of the system where the relative position in height of the left sensor-equipped support in the form of a bar (51) can be seen.

In light of this description and figures, a person skilled in the art will be able to understand that the invention has been described according to some preferred embodiments of the same, but multiple variations may be introduced in said preferred embodiments, without detracting from the object of the invention as claimed.

In this text, the term "comprise" and its derivations (such as "comprising", etc.) should not be understood in an exclusive sense. That is, these terms should not be interpreted as excluding the possibility that what is described and defined may include more elements, steps, etc.

## Claims

1. A system (1) for diagnosing the balance of a user or patient, comprising a sensor-equipped platform (2, 21), a U-shaped support structure (3) and at least three linear actuators (A1, A2, A3), **characterized in that** the U-shaped support structure (3) partially surrounds the platform (2, 21) allowing a user access to said platform (2, 21), and **in that** said structure has guiding elements (70) for guiding the mobile ends (211, 221, 231) of said linear actuators (A1, A2, A3) that allow the vertical displacement of said mobile ends (211, 221, 231) with respect to the structure (3), said mobile ends (211, 221, 231) being mechanically connected to the platform (2, 21) such that height and inclination of the platform are adjustable by vertical displacement of said mobile ends (211, 221, 231).

2. The system according to claim 1, **characterized in that** the support structure (3) has a lower support surface (31) and **in that** the platform (2, 21) can descend until its lower surface is substantially in the same plane as said lower support surface (31).

3. The system according to the preceding claims, **characterized in that** one of the linear actuators (A1) is arranged on the side opposite the access to the platform (2, 21) defined by the U-shaped support structure (3), with the two other linear actuators (A2, A3) arranged on opposite sides of said U-shaped structure (3), close to the free ends of said opposite sides of said U-shaped structure, such that the actuators (A1, A2, A3) are preferably arranged at the vertices of an imaginary isosceles triangle the sides of which join the three linear actuators together.

4. The system according to any of the preceding claims, **characterized in that** the platform (2, 21) is attached to the mobile ends (211, 221, 231) of the actuators (A1, A2, A3) by means of bars (B1, B2, B3).

5. The system according to claim 4, **characterized in that** the bars (B1, B2, B3) are attached to the mobile ends (211, 221, 231) of the actuators (A1, A2, A3) by means of joints (C1, C2, C3) and to the platform (21) by means of ball-and-socket joints (R1, R2, R3).

6. The system according to claims 3-5, **characterized in that** the platform (2, 21) can rotate laterally with respect to an instantaneous axis of rotation contained in said platform (2, 21), which contains a first point contained in the ball-and-socket joint (R1) located on the side opposite the access defined by the U-shaped platform and a point belonging to said platform that is at the same distance from the ball-and-socket joints (R2, R3) located close to the free ends of the U-shaped structure.

7. The system according to any of the preceding claims, **characterized in that** the platform can perform an anteroposterior rotation when the bar B1 and the ball-and-socket joint R1 that is integral with it rotate with respect to the joint C1, said rotation causing the rotation of the platform with respect to a shaft contained in the platform and containing the ball-and-socket joints R2, R3.

8. The system according to any of the preceding claims, **characterized in that** the platform (2, 21) can move vertically.

9. The system according to claims 6-8, **characterized in that** the platform performs a movement that is made up of one or more of the movements of claims 6-8.

10. The system according to any of the preceding claims, **characterized in that** each of the actuators (A1, A2, A3) comprises a motor that moves a worm screw which, upon rotation, moves the corresponding free end (211, 221, 231) of said actuator (A1, A2, A3) vertically.

11. The system according to claim 10, **characterized in that** the motors of the actuators are controlled by a controller which, by controlling the rotation and the rotation position of the motors, controls the movement of the platform (2, 21).

12. The system according to any of the preceding claims, **characterized in that** each of the joints (C1, C2, C3) allows rotation with respect to an imaginary horizontal axis of rotation that passes through said joint and is substantially parallel to the lower support surface of the system.

13. The system according to any of the preceding claims, wherein the platform (2, 21) comprises force sensors (40, 41, 42, 43).

14. The system according to the preceding claims, **characterized in that** the system has sensor-equipped supports in the form of handles (50, 51, 52) such that the user of the system can hold on to them during the exercise in order to help maintain balance.

15. The system according to any of the preceding claims, **characterized in that** the system has a sensor-equipped support in the form of a central handle (50) and two sensor-equipped supports in the form of handles (51, 52) placed laterally with respect to the central support.

16. The system according to claims 14, 15, **characterized in that** each of the sensor-equipped supports (50, 51, 52) in the form of handles has three force sensors.

17. The system according to any of the preceding claims, **characterized in that** it comprises a U-shaped outer casing (4) that surrounds the support structure.

18. The system according to claims 14-16, **characterized in that** each of the sensor-equipped supports in the form of handles is arranged in the upper part of the part of the casing opposite the entrance defined by the U-shaped structure.

19. The system (1) according to claim 13 or according to any of claims 14 to 18 when depending on claim 13, **characterized in that** the platform (2, 21) is formed by a first upper plate (2) and a second lower plate (21) such that the upper plate (2) is superimposed on the lower plate (21) and the force sensors are arranged between the upper plate (2) and the lower plate (21).

20. The system according to claim 19, **characterized in that** the force sensors are single-axis sensors based on strain gauges that are preferably capable of measuring forces of up to 2000 N.

21. The system according to claim 5 and any of claims 19 and 20, **characterized in that** the surface of the upper plate (2) is different from the surface of the lower plate (21) such that the ball-and-socket joints are arranged on the lower plate (21) and can protrude from it without coming into contact with the upper plate (2); and the ball-and-socket joints can be directly connected with the bars (B1, B2, B3).

## Patentansprüche

1. Ein System (1) zur Diagnose des Gleichgewichts eines Benutzers oder Patienten, umfassend eine mit Sensoren ausgestattete Plattform (2, 21), eine U-förmige Stützstruktur (3) und mindestens lineare Aktuatoren (A1, A2, A3), **dadurch gekennzeichnet, dass** die U-förmige Stützstruktur (3) die Plattform (2, 21) teilweise umgibt, sodass ein Benutzer Zugang zu der Plattform (2, 21) hat, und dass die Struktur Führungselemente (70) zum Führen der beweglichen Enden (211, 221, 231) der Linearantriebe (A1, A2, A3) aufweist, die die vertikale Verschiebung der beweglichen Enden (211, 221, 231) in Bezug auf die Struktur (3) ermöglichen, wobei die beweglichen Enden (211, 221, 231) mechanisch mit der Plattform (2, 21) verbunden sind, so dass Höhe und Neigung der Plattform durch vertikale Verschiebung der beweglichen Enden (211, 221, 231) einstellbar sind.

2. Das System gemäß Anspruch 1, **dadurch gekennzeichnet, dass** die Stützstruktur (3) eine untere Stützfläche (31) aufweist und dass die Plattform (2, 21) so weit abgesenkt werden kann, bis ihre Unterseite im Wesentlichen in derselben Ebene wie die untere Stützfläche (31) liegt.

3. Das System gemäß den vorherigen Ansprüchen, **dadurch gekennzeichnet, dass** einer der Linearantriebe (A1) auf der dem Zugang zur Plattform (2, 21) gegenüberliegenden Seite angeordnet ist, die durch die U-förmige Stützstruktur (3) definiert ist, wobei die beiden anderen Linearantriebe (A2, A3) auf gegenüberliegenden Seiten der U-förmigen Struktur (3) angeordnet sind, in der Nähe der freien Enden der gegenüberliegenden Seiten der U-förmigen Struktur angeordnet sind, so dass die Aktuatoren (A1, A2, A3) vorzugsweise an den Eckpunkten eines imaginären gleichschenkligen Dreiecks angeordnet sind, dessen Seiten die drei Linearaktuatoren miteinander verbinden.

4. Das System gemäß einem der vorherigen Ansprüche, **dadurch gekennzeichnet, dass** die Plattform (2, 21) mittels Stangen (B1, B2, B3) an den beweglichen Enden (211, 221, 231) der Aktuatoren (A1, A2, A3) befestigt ist.

5. Das System gemäß Anspruch 4, **dadurch gekennzeichnet, dass** die Stangen (B1, B2, B3) an den beweglichen Enden (211, 221, 231) der Aktuatoren (A1, A2, A3) mittels Gelenken (C1, C2, C3) und an der Plattform (21) mittels Kugelgelenken (R1, R2, R3) befestigt sind.

6. Das System gemäß den Ansprüchen 3-5, **dadurch gekennzeichnet, dass** die Plattform (2, 21) seitlich um eine in der Plattform (2, 21) enthaltene momentane Drehachse drehbar ist, die einen ersten Punkt enthält, der in dem Kugelgelenk (R1) enthalten ist, der sich auf der dem Zugang gegenüberliegenden Seite der U-förmigen Plattform befindet, und einem Punkt auf dieser Plattform, der den gleichen Abstand zu den Kugelgelenken (R2, R3) hat, die sich in der Nähe der freien Enden der U-förmigen Struktur befinden.

7. Das System nach einem der vorherigen Ansprüche, **dadurch gekennzeichnet, dass** die Plattform eine anterior-posteriore Drehung ausführen kann, wenn sich die Stange B1 und das mit ihr verbundene Kugelgelenk R1 in Bezug auf das Gelenk C1 drehen, wobei diese Drehung die Drehung der Plattform in Bezug auf eine in der Plattform enthaltene Achse bewirkt, die die Kugelgelenke R2, R3 enthält.

8. Das System gemäß einem der vorherigen Ansprüche, **dadurch gekennzeichnet, dass** sich die Plattform (2, 21) vertikal bewegen kann.

9. Das System gemäß den Ansprüchen 6-8, **dadurch gekennzeichnet, dass** die Plattform eine Bewegung ausführt, die aus einer oder mehreren der Bewegungen der Ansprüche 6-8 besteht.

10. Das System gemäß einem der vorherigen Ansprüche, **dadurch gekennzeichnet, dass** jeder der Aktuatoren (A1, A2, A3) einen Motor umfasst, der eine Schneckenschraube bewegt, die bei Drehung das entsprechende freie Ende (211, 221, 231) des Aktuators (A1, A2, A3) vertikal bewegt.

11. Das System gemäß Anspruch 10, **dadurch gekennzeichnet, dass** die Motoren der Aktuatoren von einer Steuereinheit gesteuert werden, die durch Steuerung der Drehung und der Drehposition der Motoren die Bewegung der Plattform (2, 21) steuert.

12. Das System gemäß einem der vorherigen Ansprüche, **dadurch gekennzeichnet, dass** jedes der Gelenke (C1, C2, C3) eine Drehung um eine imaginäre horizontale Drehachse ermöglicht, die durch das Gelenk verläuft und im Wesentlichen parallel zur unteren Auflagefläche des Systems ist.

13. Das System gemäß einem der vorherigen Ansprüche, wobei die Plattform (2, 21) Kraftsensoren (40, 41, 42, 43) umfasst.

14. Das System gemäß den vorherigen Ansprüchen, **dadurch gekennzeichnet, dass** das System mit Sensoren ausgestattete Stützen in Form von Griffen (50, 51, 52) aufweist, so dass der Benutzer des Systems sich während der Übung daran festhalten kann, um das Gleichgewicht zu halten.

15. Das System gemäß einem der vorherigen Ansprüche, **dadurch gekennzeichnet, dass** das System eine mit Sensoren ausgestattete Stütze in Form eines zentralen Griffs (50) und zwei mit Sensoren ausgestattete Stützen in Form von Griffen (51, 52) aufweist, die seitlich in Bezug auf die zentrale Stütze angeordnet sind.

16. Das System gemäß den Ansprüchen 14, 15, **dadurch gekennzeichnet, dass** jede der mit Sensoren ausgestatteten Stützen (50, 51, 52) in Form von Griffen drei Kraftsensoren aufweist.

17. Das System gemäß einem der vorherigen Ansprüche, **dadurch gekennzeichnet, dass** es ein U-förmiges Außengehäuse (4) umfasst, das die Stützstruktur umgibt.

18. Das System gemäß den Ansprüchen 14-16, **dadurch gekennzeichnet, dass** jede der mit Sensoren ausgestatteten Stützen in Form von Griffen im oberen Teil des Teils des Gehäuses angeordnet ist, der dem durch die U-förmige Struktur definierten Eingang gegenüberliegt.

19. Das System (1) gemäß Anspruch 13 oder gemäß einem der Ansprüche 14 bis 18, wenn es von Anspruch 13 abhängt, **dadurch gekennzeichnet, dass** die Plattform (2, 21) durch eine erste obere Platte (2) und eine zweite untere Platte (21) gebildet ist, so dass die obere Platte (2) auf der unteren Platte (21) aufliegt und die Kraftsensoren zwischen der oberen Platte (2) und der unteren Platte (21) angeordnet sind.

20. Das System gemäß Anspruch 19, **dadurch gekennzeichnet, dass** die Kraftsensoren einachsige Sensoren auf Basis von Dehnungsmessstreifen sind, die vorzugsweise Kräfte von bis zu 2000 N messen können.

21. Das System gemäß Anspruch 5 und einem der Ansprüche 19 und 20, **dadurch gekennzeichnet, dass** sich die Oberfläche der oberen Platte (2) von der Oberfläche der unteren Platte (21) unterscheidet, so dass die Kugelgelenke an der unteren Platte (21) angeordnet sind und aus dieser herausragen können, ohne mit der oberen Platte (2) in Kontakt zu kommen; und die Kugelgelenke direkt mit den Stangen (B1, B2, B3) verbunden werden können.

## Revendications

1. Système (1) pour diagnostiquer l'équilibre d'un utilisateur ou d'un patient, comprenant une plateforme équipée de capteurs (2, 21), une structure de support en forme de U (3) et au moins trois actionneurs linéaires (A1, A2, A3), **caractérisé en ce que** la structure de support en forme de U (3) entoure partiellement la plateforme (2, 21) permettant à un utilisateur d'accéder à ladite plateforme (2, 21), et **en ce que** ladite structure comporte des éléments de guidage (70) pour guider les extrémités mobiles (211, 221, 231) desdits actionneurs linéaires (A1, A2, A3) et qui permettent le déplacement vertical desdites extrémités mobiles (211, 221, 231) par rapport à la structure (3), lesdites extrémités mobiles (211, 221, 231) étant reliées mécaniquement à la plateforme (2, 21) de sorte que la hauteur et l'inclinaison de la plateforme sont réglables par déplacement vertical desdites extrémités mobiles (211, 221, 231).

2. Système selon la revendication 1, **caractérisé en ce que** la structure de support (3) comporte une surface de support inférieure (31), et **en ce que** la plateforme (2, 21) peut descendre jusqu'à ce que sa surface inférieure soit sensiblement dans le même plan que ladite surface de support inférieure (31).

3. Système selon les revendications précédentes, **caractérisé en ce que** l'un des actionneurs linéaires (A1) est disposé du côté opposé à l'accès à la plateforme (2, 21) défini par la structure de support en forme de U (3), les deux autres actionneurs linéaires (A2, A3) étant disposés sur des côtés opposés de ladite structure en forme de U (3), à proximité des extrémités libres desdits côtés opposés de ladite structure en forme de U, de sorte que les actionneurs (A1, A2, A3) sont de préférence disposés aux sommets d'un triangle isocèle imaginaire dont les côtés relient ensemble les trois actionneurs linéaires.

4. Système selon l'une quelconque des revendications précédentes, **caractérisé en ce que** la plateforme (2, 21) est fixée aux extrémités mobiles (211, 221, 231) des actionneurs (A1, A2, A3) au moyen de barres (B1, B2, B3).

5. Système selon la revendication 4, **caractérisé en ce que** les barres (B1, B2, B3) sont fixées aux extrémités mobiles (211, 221, 231) des actionneurs (A1, A2, A3) au moyen d'articulations (C1, C2, C3) et à la plateforme (21) au moyen d'articulations à rotule (R1, R2, R3).

6. Système selon les revendications 3 à 5, **caractérisé en ce que** la plateforme (2, 21) peut effectuer une rotation latérale par rapport à un axe instantané de rotation contenu dans ladite plateforme (2, 21), lequel contient un premier point contenu dans l'articulation à rotule (R1) située du côté opposé à l'accès défini par la plateforme en forme de U et un point appartenant à ladite plateforme qui est à la même distance des articulations à rotule (R2, R3) situées à proximité des extrémités libres de la structure en forme de U.

7. Système selon l'une quelconque des revendications précédentes, **caractérisé en ce que** la plateforme peut effectuer une rotation antéro-postérieure lorsque la barre B1 et l'articulation à rotule R1 qui lui est solidaire tournent par rapport à l'articulation C1, ladite rotation provoquant la rotation de la plateforme par rapport à un axe contenu dans la plateforme et contenant les articulations à rotule R2, R3.

8. Système selon l'une quelconque des revendications précédentes, **caractérisé en ce que** la plateforme (2, 21) peut se déplacer verticalement.

9. Système selon les revendications 6 à 8, **caractérisé en ce que** la plateforme effectue un mouvement constitué d'un ou de plusieurs des mouvements des revendications 6 à 8.

10. Système selon l'une quelconque des revendications précédentes, **caractérisé en ce que** chacun des actionneurs (A1, A2, A3) comprend un moteur qui entraîne une vis sans fin qui, en tournant, déplace verticalement l'extrémité libre (211, 221, 231) correspondante dudit actionneur (A1, A2, A3).

11. Système selon la revendication 10, **caractérisé en ce que** les moteurs des actionneurs sont commandés par un contrôleur qui commande le mouvement de la plateforme (2, 21) en commandant la rotation et la position de rotation des moteurs.

12. Système selon l'une quelconque des revendications précédentes, **caractérisé en ce que** chacune des articulations (C1, C2, C3) permet une rotation par rapport à un axe de rotation horizontal imaginaire qui passe par ladite articulation et qui est sensiblement parallèle à la surface de support inférieure du système.

13. Système selon l'une quelconque des revendications précédentes, dans lequel la plateforme (2, 21) comprend des capteurs de force (40, 41, 42, 43).

14. Système selon les revendications précédentes, **caractérisé en ce que** le système comporte des supports équipés de capteurs sous la forme de poignées (50, 51, 52) de sorte que l'utilisateur du système peut s'y tenir pendant l'exercice afin de l'aider à maintenir l'équilibre.

15. Système selon l'une quelconque des revendications précédentes, **caractérisé en ce que** le système comporte un support équipé de capteurs sous la forme d'une poignée centrale (50) et deux supports équipés de capteurs sous la forme de poignées (51, 52) placées latéralement par rapport au support central.

16. Système selon les revendications 14, 15, **caractérisé en ce que** chacun des supports équipés de capteurs (50, 51, 52) sous la forme de poignées comporte trois capteurs de force.

17. Système selon l'une quelconque des revendications précédentes, **caractérisé en ce qu'il** comprend un carter extérieur en forme de U (4) qui entoure la structure de support.

18. Système selon les revendications 14 à 16, **caractérisé en ce que** chacun des supports équipés de capteurs sous la forme de poignées est disposé dans la partie supérieure de la partie du carter opposée à l'entrée définie par la structure en forme de U.

19. Système (1) selon la revendication 13 ou selon l'une quelconque des revendications 14 à 18 lorsqu'elles dépendent de la revendication 13, **caractérisé en ce que** la plateforme (2, 21) est formée par une première plaque supérieure (2) et une seconde plaque inférieure (21) de sorte que la plaque supérieure (2) est superposée à la plaque inférieure (21) et que les capteurs de force sont disposés entre la plaque supérieure (2) et la plaque inférieure (21).

20. Système selon la revendication 19, **caractérisé en ce que** les capteurs de force sont des capteurs uniaxiaux basés sur des jauges de contrainte qui sont de préférence aptes à mesurer des forces allant jusqu'à 2 000 N.

21. Système selon la revendication 5 et l'une quelconque des revendications 19 et 20, **caractérisé en ce que** la surface de la plaque supérieure (2) est différente de la surface de la plaque inférieure (21) de sorte que les articulations à rotule sont disposées sur la plaque inférieure (21) et peuvent en saillir sans entrer en contact avec la plaque supérieure (2); et les articulations à rotule peuvent être directement reliées aux barres (B1, B2, B3).
